# EUROPEAN PATENT APPLICATION

(11) **EP 4 781 987 A1**
(43) Date of publication of application: **29.07.2026**
(21) Application number: 24867489.7
(22) Date of filing: 19.09.2024
(51) Int. Cl.: A61K 31/454, A61K 31/439, A61P 13/12

(54) **USE OF HETEROCYCLIC COMPOUND IN PREPARATION OF DRUG FOR PREVENTING AND/OR TREATING KIDNEY DISEASE**

(30) Priority: 20.09.2023 CN 202311217283; 20.09.2023 CN 202311217299; 12.09.2024 CN 202411281982
(71) Applicant: Shanghai Meiyue Biotech Development Co., Ltd., Shanghai 200120 (CN)
(72) Inventor: LUAN, Linbo, Shanghai 200120 (CN); TANG, Chunlan, Shanghai 200120 (CN); XIA, Rui, Shanghai 200120 (CN)
(74) Representative: IK-IP LTD
(86) International application number: PCT/CN2024/119619
(87) International publication number: WO 2025/061083

(57) **Abstract**

A use of a compound as shown in formula (I) or a pharmaceutically acceptable salt thereof in preparation of a drug for treating and/or preventing a kidney disease, wherein the compound of formula (I) is as follows: (I).

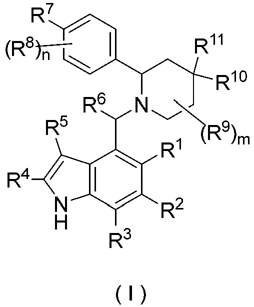

## Description

The present application claims priority to the prior application filed with the China National Intellectual Property Administration on September 20, 2023, with the patent application number of 202311217283.0, the prior application filed with the China National Intellectual Property Administration on September 20, 2023, with the patent application number of 202311217299.1, and the prior application filed with the China National Intellectual Property Administration on September 12, 2024, with the patent application number of 202411281982.6. The contents of the above prior applications are incorporated herein by reference in their entireties.

### TECHNICAL FIELD

The present disclosure belongs to the field of medicine, and specifically relates to the use of a heterocyclic compound in the manufacture of a medicament for preventing and/or treating a kidney disease.

### BACKGROUND

Diabetic kidney disease (DKD), also known as diabetic nephropathy, is a chronic kidney disease (CKD) caused by diabetes mellitus (DM), with a complex pathogenesis. Clinically, DKD is characterized by a persistent increase in urinary albumin excretion and/or a progressive decline in glomerular filtration rate (GFR), ultimately progressing to end-stage renal disease (ESRD).

DKD is mainly defined by a urinary albumin-to-creatinine ratio (UACR) of 30 mg/g or more or a urinary albumin excretion rate (UAER) of 30 mg/24 h or more, with repeated measurements of UACR or UAER performed within 3 to 6 months, in which two out of three results reach or exceed the threshold, after excluding other interfering factors such as infection; and/or an estimated glomerular filtration rate (eGFR) of less than 60 mL·min⁻¹·(1.73 m²)⁻¹ that persists for more than 3 months. DKD is renal damage caused by chronic hyperglycemia, and the lesions may involve the entire kidney (including glomeruli, renal tubules, renal interstitium, and renal vasculature). Podocytes are located on the epithelial surface of the glomeruli, through which molecules below 60 kDa are filtered. The effacement of podocyte foot processes is associated with the occurrence of proteinuria and nephrotic syndrome. A decrease in the number of podocyte foot processes and podocyte hypertrophy are observed in early-stage DKD, while loss of podocytes is observed in late-stage DKD. Late-stage DKD is typically accompanied by massive, often nephrotic-range proteinuria, highlighting the critical role of podocytes in DKD. Podocytes are terminally differentiated cells that are incapable of proliferation. Loss of more than 20% of podocytes represents an irreversible step in the pathogenesis of DKD, leading to glomerular scarring and the progression to end-stage renal disease.

According to the 2021 *Chinese Guidelines for the Clinical Diagnosis and Treatment of Diabetic Kidney Disease*, existing treatments for diabetic kidney disease can be summarized as follows: lifestyle management, glycemic control, control of blood pressure and urinary protein, regulation of blood lipid levels, and control of uric acid levels. However, existing treatments for DKD lack high-quality evidence-based medical data to demonstrate their efficacy. Developing new and effective therapeutics to meet the clinical needs of patients with DKD holds significant social and practical importance.

Lupus nephritis (LN) is the most common and significant renal complication of systemic lupus erythematosus (SLE), with a relatively complex pathogenesis. SLE is the most common systemic autoimmune disease in China. The kidneys are the organs most frequently affected by SLE.

LN presents with diverse clinical manifestations, ranging from asymptomatic proteinuria and/or hematuria in mild cases to nephrotic syndrome or rapidly progressive glomerulonephritis in severe cases. Persistent and recurrent lesions may also lead to chronic renal insufficiency or even renal failure. Patients may exhibit abnormal urine output (oliguria or nocturia), hematuria, foamy urine, edema, and hypertension. Microscopic hematuria is present in most cases. Proteinuria is the most common manifestation, with varying degrees of severity. Patients may develop elevated blood pressure, which is more pronounced when accompanied by renal vascular lesions.

### SUMMARY

To address the aforementioned technical issues, the present disclosure provides the use of a heterocyclic compound in the manufacture of a medicament for treating and/or preventing a kidney disease, wherein the kidney disease is diabetic nephropathy or lupus nephritis.

The present disclosure provides the use of a compound represented by formula (I) or a pharmaceutically acceptable salt thereof in the manufacture of a medicament for treating and/or preventing a kidney disease, wherein the kidney disease is diabetic nephropathy or lupus nephritis, 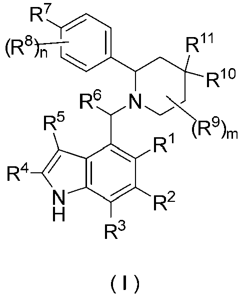 wherein
R¹ is selected from the group consisting of halogen, OH, CN, C₁₋₆ alkyl, C₃₋₁₀ cycloalkyl, C₁₋₆ alkoxy, C₁₋₆ deuteroalkoxy, C₃₋₁₀ cycloalkyloxy, C₁₋₆ haloalkyl, and C₁₋₆ haloalkoxy;
R² is selected from the group consisting of H, halogen, OH, CN, C₁₋₆ alkyl, C₃₋₁₀ cycloalkyl, C₁₋₆ alkoxy, C₁₋₆ haloalkyl, and C₁₋₆ haloalkoxy;
R³ is selected from the group consisting of H, halogen, OH, CN, C₁₋₆ alkyl, C₁₋₆ deuteroalkyl, C₃₋₁₀ cycloalkyl, C₁₋₆ alkoxy, C₃₋₁₀ cycloalkyloxy, NH₂, C₁₋₆ haloalkyl, and C₁₋₆ haloalkoxy;
R⁴ is selected from the group consisting of H, C₁₋₆ alkyl, and C₁₋₆ haloalkyl;
R⁵ is selected from the group consisting of H, halogen, and C₁₋₆ alkyl;
R⁶ is selected from the group consisting of H, halogen, OH, CN, and C₁₋₆ alkyl;
R⁷ is selected from the group consisting of -COOH and 5-membered heteroaryl;
R⁸ is selected from the group consisting of H, halogen, OH, CN, and C₁₋₆ alkyl;
R⁹ is selected from the group consisting of H, halogen, OH, CN, C₁₋₆ alkyl, and C₁₋₆ haloalkyl; or any two R⁹ together form a C₁₋₆ alkylene group;
R¹⁰ and R¹¹ are the same or different, and each independently selected from the group consisting of H, halogen, OH, CN, C₁₋₆ alkyl, C₃₋₁₀ cycloalkyl, 3- to 10-membered heterocyclyl, C₁₋₆ alkoxy, C₃₋₁₀ cycloalkyloxy, and 3- to 10-membered heterocyclyloxy, wherein the C₁₋₆ alkyl, C₃₋₁₀ cycloalkyl, 3- to 10-membered heterocyclyl, C₁₋₆ alkoxy, C₃₋₁₀ cycloalkyloxy, and 3- to 10-membered heterocyclyloxy are optionally substituted by one or more R^{a};
R^{a} is selected from the group consisting of halogen, OH, CN, C₁₋₆ alkyl, C₃₋₁₀ cycloalkyl, 3- to 10-membered heterocyclyl, and C₁₋₆ alkoxy, wherein the C₁₋₆ alkyl, C₃₋₁₀ cycloalkyl, 3- to 10-membered heterocyclyl, and C₁₋₆ alkoxy are optionally substituted by one or more substituents selected from the group consisting of halogen, OH, CN, and C₁₋₆ alkyl;
n is selected from the group consisting of 0, 1, 2, 3, and 4;
m is selected from the group consisting of 0, 1, 2, 3, 4, 5, and 6.

In some embodiments of the present disclosure, R¹⁰ is selected from the group consisting of H, halogen, C₁₋₆ alkyl, C₃₋₆ cycloalkyl, 3- to 6-membered heterocyclyl, C₁₋₆ alkoxy, C₃₋₆ cycloalkyloxy, and 3- to 6-membered heterocyclyloxy, wherein the C₁₋₆ alkyl, C₃₋₆ cycloalkyl, 3- to 6-membered heterocyclyl, C₁₋₆ alkoxy, C₃₋₆ cycloalkyloxy, and 3- to 6-membered heterocyclyloxy are optionally substituted by one or more R^{a};
R¹¹ is selected from the group consisting of H, halogen, and C₁₋₆ alkyl;
R^{a} is selected from the group consisting of halogen, C₁₋₆ alkyl, C₃₋₆ cycloalkyl, and 3-to 6-membered heterocyclyl, wherein the C₁₋₆ alkyl, C₃₋₆ cycloalkyl, and 3- to 6-membered heterocyclyl are optionally substituted by one or more substituents selected from the group consisting of halogen, OH, CN, and C₁₋₆ alkyl.

In some embodiments of the present disclosure, the groups in the compound represented by formula (I) are defined as follows:
R¹ is C₁₋₆ alkoxy; R² is H; R³ is selected from the group consisting of C₁₋₆ alkyl, C₁₋₆ deuteroalkyl, and C₃₋₁₀ cycloalkyl; R⁴ is H; R⁵ is H or halogen; R⁶ is H; R⁷ is -COOH; R⁸ is selected from the group consisting of H, halogen, and C₁₋₆ alkyl; R⁹ is selected from the group consisting of H, halogen, and C₁₋₆ alkyl; R¹⁰ is selected from the group consisting of C₁₋₆ alkoxy, C₃₋₆ cycloalkyloxy, and 3- to 6-membered heterocyclyloxy, wherein the C₁₋₆ alkoxy, C₃₋₆ cycloalkyloxy, and 3- to 6-membered heterocyclyloxy are optionally substituted by one or more R^{a}; R¹¹ is H; R^{a} is selected from the group consisting of halogen, C₃₋₆ cycloalkyl, and 3-to 6-membered heterocyclyl, wherein the C₃₋₆ cycloalkyl and 3- to 6-membered heterocyclyl are optionally substituted by one or more substituents selected from the group consisting of halogen, OH, CN, and C₁₋₆ alkyl; n is selected from the group consisting of 0, 1, and 2; m is selected from the group consisting of 0, 1, and 2. In some embodiments of the present disclosure, the groups in the compound represented by formula (I) are defined as follows:
R¹ is C₁₋₆ alkoxy; R² is H; R³ is selected from the group consisting of C₁₋₆ alkyl, C₁₋₆ deuteroalkyl, and C₃₋₆ cycloalkyl; R⁴ is H; R⁵ is H or halogen; R⁶ is H; R⁷ is -COOH; R⁸ is selected from the group consisting of H, halogen, and C₁₋₆ alkyl; R⁹ is selected from the group consisting of H, halogen, and C₁₋₆ alkyl; R¹⁰ is selected from the group consisting of C₃₋₆ cycloalkyl and 3- to 6-membered heterocyclyl, wherein the C₃₋₆ cycloalkyl and 3- to 6-membered heterocyclyl are optionally substituted by one or more R^{a}; R¹¹ is H; R^{a} is halogen or C₁₋₆ alkyl; n is selected from the group consisting of 0, 1, and 2; m is selected from the group consisting of 0, 1, and 2.

In some embodiments of the present disclosure, the groups in the compound represented by formula (I) are defined as follows:
R¹ is C₁₋₆ alkoxy; R² is H; R³ is selected from the group consisting of C₁₋₆ alkyl, C₁₋₆ deuteroalkyl, and C₃₋₆ cycloalkyl; R⁴ is H; R⁵ is H or halogen; R⁶ is H; R⁷ is -COOH; R⁸ is selected from the group consisting of H, halogen, and C₁₋₆ alkyl; any two R⁹ together form a - CH₂- or -CH₂CH₂- group; R¹⁰ is selected from the group consisting of H, halogen, C₁₋₆ alkyl, C₁₋₆ alkoxy, C₃₋₆ cycloalkyl, and 3- to 6-membered heterocyclyl, wherein the C₁₋₆ alkyl, C₁₋₆ alkoxy, C₃₋₆ cycloalkyl, and 3- to 6-membered heterocyclyl are optionally substituted by one or more R^{a}; R¹¹ is H, halogen, or C₁₋₆ alkyl; R^{a} is selected from the group consisting of halogen, C₁₋₆ alkyl, C₃₋₆ cycloalkyl, and 3- to 6-membered heterocyclyl; n is selected from the group consisting of 0, 1, 2, 3, and 4; m is 2.

In some embodiments of the present disclosure, the compound represented by formula (I) is a compound represented by formula (II) as follows, 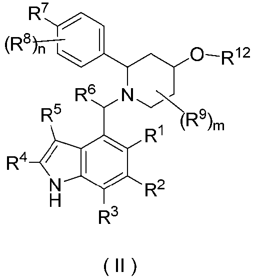 wherein R⁹ is selected from the group consisting of H, halogen, and C₁₋₆ alkyl; R¹² is selected from the group consisting of C₁₋₆ alkyl, C₃₋₆ cycloalkyl, and 3- to 6-membered heterocyclyl, wherein the C₁₋₆ alkyl, C₃₋₆ cycloalkyl, and 3- to 6-membered heterocyclyl are optionally substituted by one or more R^{a}; R^{a} is selected from the group consisting of halogen, C₃₋₆ cycloalkyl, and 3- to 6-membered heterocyclyl, wherein the C₃₋₆ cycloalkyl and 3- to 6-membered heterocyclyl are optionally substituted by one or more substituents selected from the group consisting of halogen, OH, CN, and C₁₋₆ alkyl; R¹ to R⁸, n, and m are as defined in the compound represented by formula (I) described above.

In some embodiments of the present disclosure, the groups in the compound represented by formula (II) are defined as follows:
R¹ is C₁₋₆ alkoxy; R² is H; R³ is C₁₋₆ alkyl; R⁴ is H; R⁵ is H or halogen; R⁶ is H; R⁷ is -COOH; R⁸ is selected from the group consisting of H, halogen, and C₁₋₆ alkyl; R⁹ is selected from the group consisting of H, halogen, and C₁₋₆ alkyl; R¹² is C₁₋₆ alkyl, wherein the C₁₋₆ alkyl is optionally substituted by one or more R^{a}; R^{a} is selected from the group consisting of halogen, C₃₋₆ cycloalkyl, and 3- to 6-membered heterocyclyl, wherein the C₃₋₆ cycloalkyl and 3- to 6-membered heterocyclyl are optionally substituted by one or more substituents selected from the group consisting of halogen, OH, CN, and C₁₋₆ alkyl; n is selected from the group consisting of 0, 1, and 2; m is selected from the group consisting of 0, 1, and 2.

In some embodiments of the present disclosure, the compound represented by formula (I) is a compound represented by formula (III-1), formula (III-2), formula (III-3), or formula (III-4) as follows, 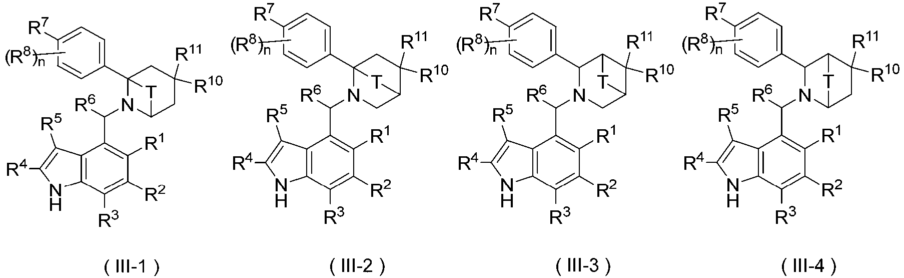 wherein T is -CH₂- or -CH₂CH₂-; R¹⁰ is selected from the group consisting of H, halogen, C₁₋₆ alkyl, C₁₋₆ alkoxy, C₃₋₆ cycloalkyl, and 3- to 6-membered heterocyclyl, wherein the C₁₋₆ alkyl, C₁₋₆ alkoxy, C₃₋₆ cycloalkyl, and 3- to 6-membered heterocyclyl are optionally substituted by one or more R^{a}; R¹¹ is H, halogen, or C₁₋₆ alkyl; R¹ to R⁸, n, and m are as defined in the compound represented by formula (I) described above.

In some embodiments of the present disclosure, the compound represented by formula (I) may be selected from the group consisting of the following structures: 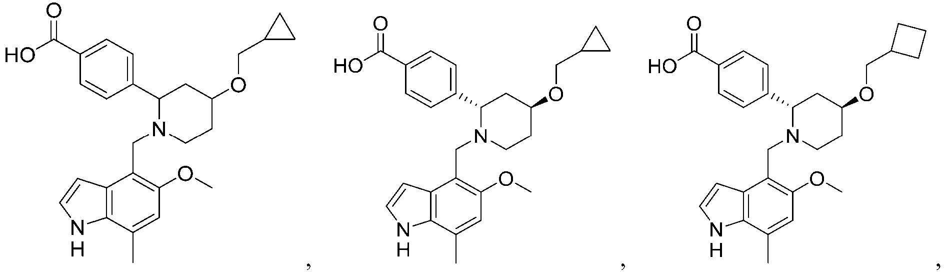 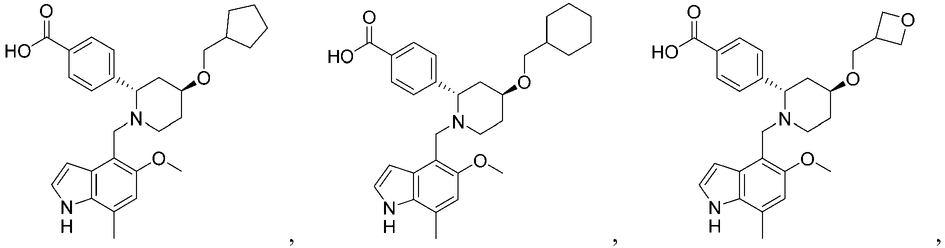 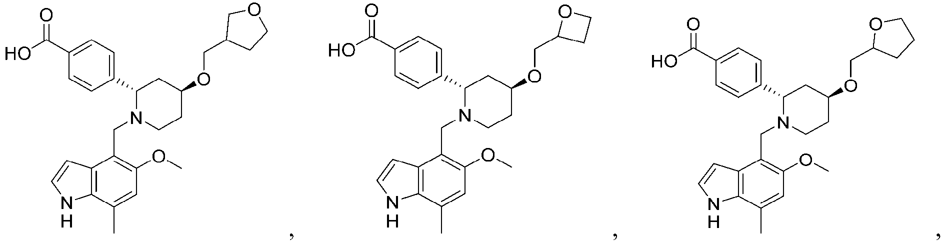 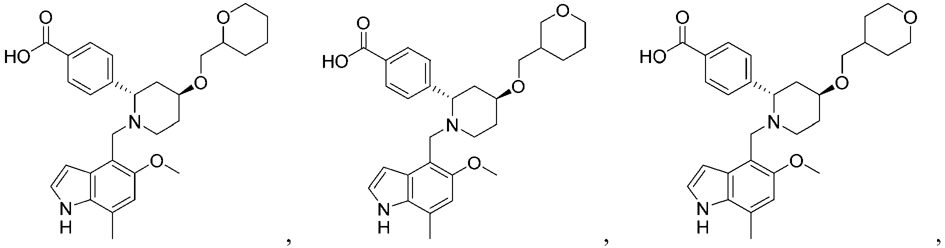 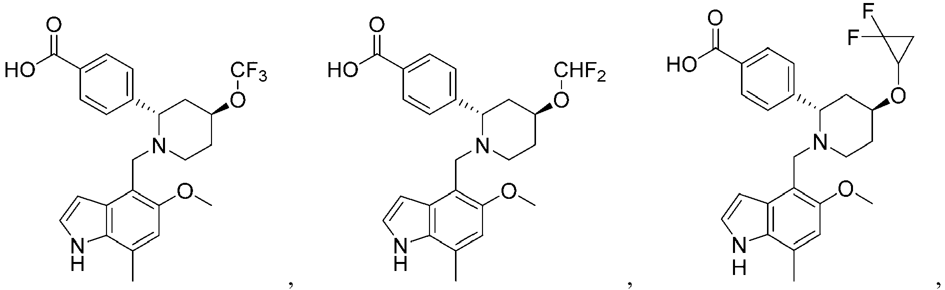 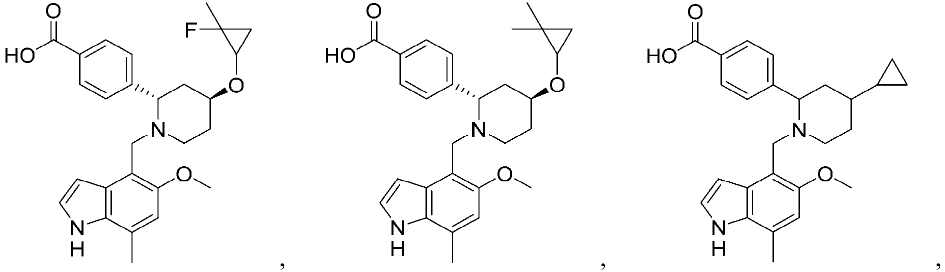 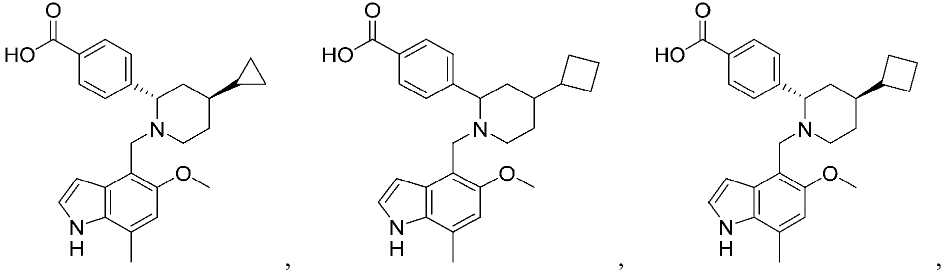 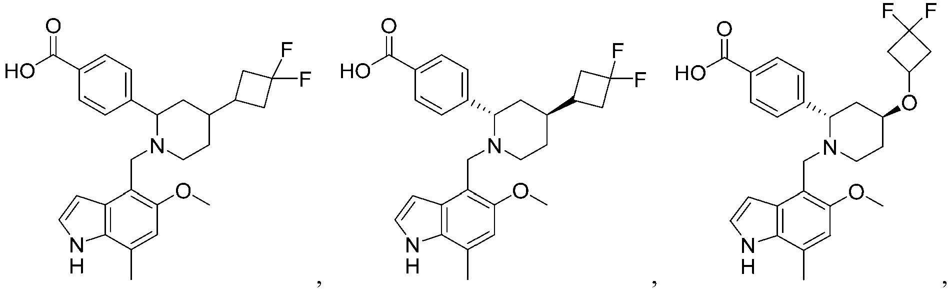 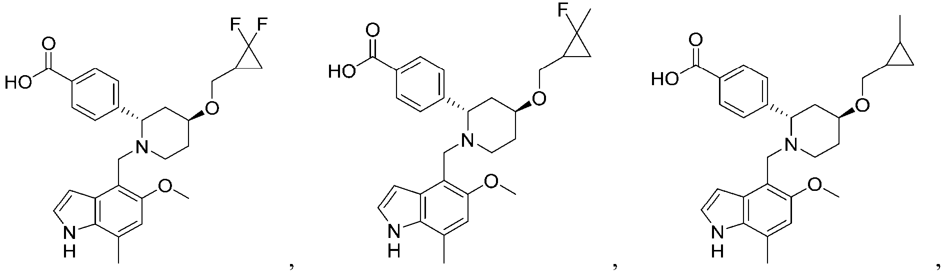 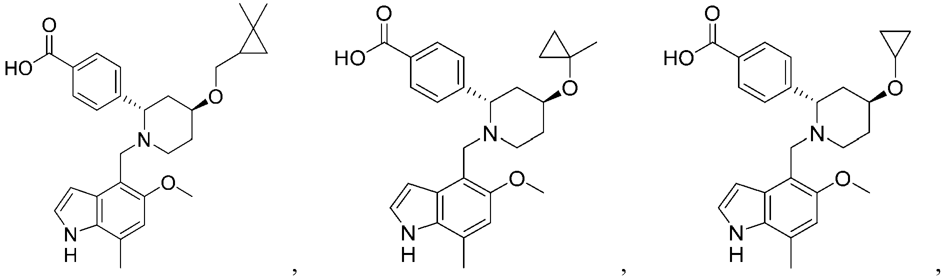 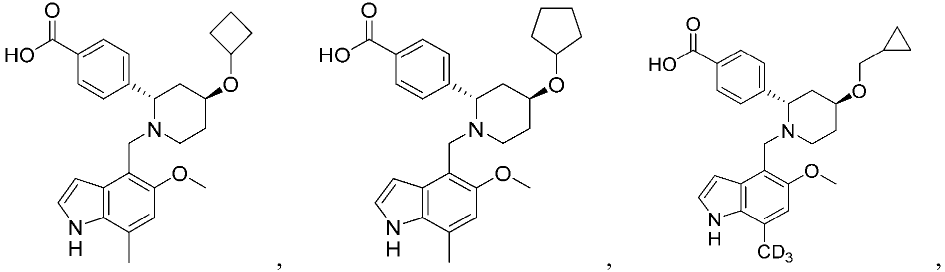 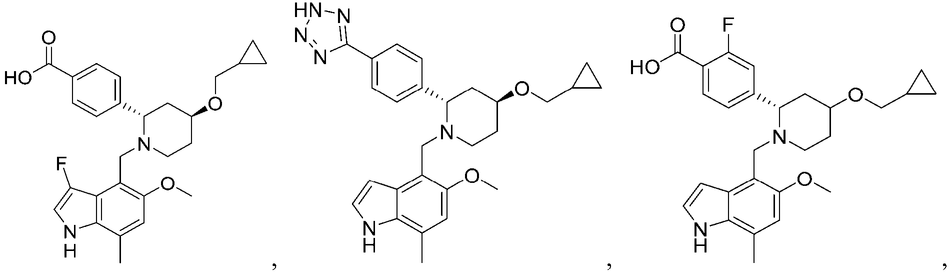 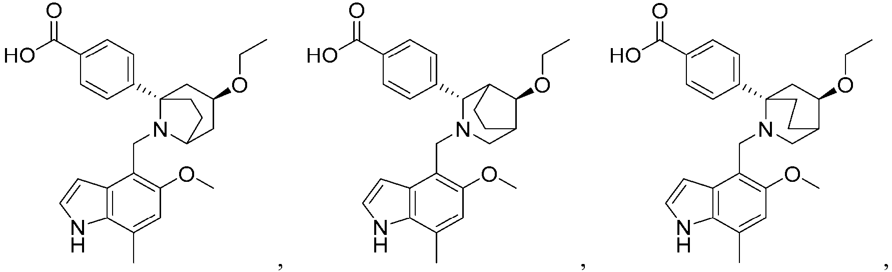 and

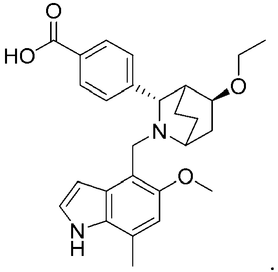

In some embodiments of the present disclosure, the compound represented by formula (I) is compound A, and the structural formula of compound A is

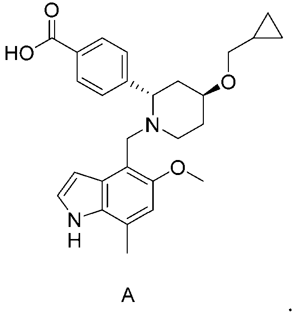

In some embodiments of the present disclosure, the pharmaceutically acceptable salt is selected from the group consisting of hydrochloride, phosphate, hydrogen phosphate, sulfate, hydrogen sulfate, sulfite, acetate, oxalate, malonate, valerate, glutamate, oleate, stearate, laurate, *p*-toluenesulfonate, methanesulfonate, fumarate, hydroxyethanesulfonate, maleate, malate, tartrate, citrate, oxalate, benzoate, pamoate, salicylate, vanillate, and succinate salts.

In some embodiments of the present disclosure, the pharmaceutically acceptable salt is a hydrochloride salt.

In some embodiments of the present disclosure, the pharmaceutically acceptable salt is a monohydrochloride salt.

In some embodiments of the present disclosure, the compound represented by formula (I) or the pharmaceutically acceptable salt thereof is compound B:

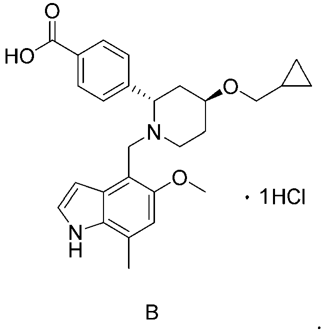

In some embodiments of the present disclosure, the kidney disease is diabetic nephropathy.

In another aspect, the present disclosure provides the use of a compound represented by formula (I) or a pharmaceutically acceptable salt thereof in the manufacture of a medicament for delaying progressive decline in renal function in a subject with diabetic nephropathy.

In another aspect, the present disclosure provides the use of a compound represented by formula (I) or a pharmaceutically acceptable salt thereof in the manufacture of a medicament for delaying progressive decline in glomerular filtration rate (GFR) in a subject with diabetic nephropathy.

In another aspect, the present disclosure provides the use of a compound represented by formula (I) or a pharmaceutically acceptable salt thereof in the manufacture of a medicament for improving a urinary protein-to-creatinine ratio (UPCR) and/or a urinary albumin-to-creatinine ratio (UACR) in a subject with diabetic nephropathy.

In some embodiments of the present disclosure, the subject suffers from type 2 diabetes.

In some embodiments of the present disclosure, the subject with diabetic nephropathy has a urinary albumin-to-creatinine ratio (UACR) of 300 mg/g or more and less than 1500 mg/g.

In some embodiments of the present disclosure, the kidney disease is lupus nephritis.

In some embodiments of the present disclosure, the lupus nephritis comprises minimal mesangial LN (Class I), mesangial proliferative LN (Class II), focal LN (Class III), diffuse LN (Class IV), membranous LN (Class V), and sclerosing LN (Class VI).

In some embodiments of the present disclosure, the lupus nephritis is focal LN (Class III) or diffuse LN (Class IV).

In some embodiments of the present disclosure, the lupus nephritis is new-onset or relapsed lupus nephritis.

In some embodiments of the present disclosure, the relapsed lupus nephritis is lupus nephritis that has relapsed after treatment with cyclophosphamide or mycophenolate mofetil (MMF).

In some embodiments of the present disclosure, the patient with lupus nephritis suffers from systemic lupus erythematosus (SLE).

In another aspect, the present disclosure provides the use of compound A or a pharmaceutically acceptable salt thereof in the manufacture of a medicament for improving a urinary protein-to-creatinine ratio (UPCR) and/or a urinary albumin-to-creatinine ratio (UACR) in a subject with lupus nephritis.

In some embodiments of the present disclosure, the medicament is administered in an oral dosage form or an injectable dosage form.

In some embodiments of the present disclosure, the medicament is administered in an oral dosage form.

In some embodiments of the present disclosure, the medicament is administered at a dose (calculated based on the active ingredient) of any value within a range of 0.1 to 1000 mg, or 0.1 to 800 mg, or 100 to 800 mg, or 200 to 600 mg, or any range formed by a combination of any values within the range, such as 10 mg, 50 mg, 100 mg, 150 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 305 mg, 310 mg, 315 mg, 320 mg, 325 mg, 330 mg, 335 mg, 340 mg, 345 mg, 350 mg, 355 mg, 360 mg, 365 mg, 370 mg, 375 mg, 380 mg, 385 mg, 390 mg, 395 mg, 400 mg, 405 mg, 410 mg, 415 mg, 420 mg, 425 mg, 430 mg, 435 mg, 440 mg, 445 mg, 450 mg, 455 mg, 460 mg, 465 mg, 470 mg, 475 mg, 480 mg, 485 mg, 490 mg, 495 mg, 500 mg, 505 mg, 510 mg, 515 mg, 520 mg, 525 mg, 530 mg, 535 mg, 540 mg, 545 mg, 550 mg, 555 mg, 560 mg, 565 mg, 570 mg, 575 mg, 580 mg, 585 mg, 590 mg, 595 mg, 600 mg, 705 mg, 710 mg, 715 mg, 720 mg, 725 mg, 730 mg, 735 mg, 740 mg, 745 mg, 750 mg, 755 mg, 760 mg, 765 mg, 770 mg, 775 mg, 780 mg, 785 mg, 790 mg, 795 mg, 800 mg, 850 mg, 900 mg, 950 mg, or 1000 mg.

In some embodiments of the present disclosure, the medicament is administered at a dose (calculated based on the active ingredient) of any value within a range of 0.1 mg/kg to 300 mg/kg, preferably 4 mg/kg to 20 mg/kg, or any range formed by a combination of any values within the range, such as 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg, 21 mg/kg, 22 mg/kg, 23 mg/kg, 24 mg/kg, 25 mg/kg, 26 mg/kg, 27 mg/kg, 28 mg/kg, 29 mg/kg, 30 mg/kg, 31 mg/kg, 32 mg/kg, 33 mg/kg, 34 mg/kg, 35 mg/kg, 36 mg/kg, 37 mg/kg, 38 mg/kg, 39 mg/kg, 40 mg/kg, 41 mg/kg, 42 mg/kg, 43 mg/kg, 44 mg/kg, 45 mg/kg, 46 mg/kg, 47 mg/kg, 48 mg/kg, 49 mg/kg, 50 mg/kg, 55 mg/kg, 60 mg/kg, 65 mg/kg, 70 mg/kg, 75 mg/kg, 80 mg/kg, 85 mg/kg, 90 mg/kg, 95 mg/kg, 100 mg/kg, 105 mg/kg, 110 mg/kg, 115 mg/kg, 120 mg/kg, 125 mg/kg, 130 mg/kg, 135 mg/kg, 140 mg/kg, 145 mg/kg, 150 mg/kg, 155 mg/kg, 160 mg/kg, 165 mg/kg, 170 mg/kg, 175 mg/kg, 180 mg/kg, 185 mg/kg, 190 mg/kg, 195 mg/kg, 200 mg/kg, 205 mg/kg, 210 mg/kg, 215 mg/kg, 220 mg/kg, 225 mg/kg, 230 mg/kg, 235 mg/kg, 240 mg/kg, 245 mg/kg, 250 mg/kg, 255 mg/kg, 260 mg/kg, 265 mg/kg, 270 mg/kg, 275 mg/kg, 280 mg/kg, 285 mg/kg, 290 mg/kg, 295 mg/kg, or 300 mg/kg.

In some embodiments of the present disclosure, the dosing frequency of the medicament may be once daily, twice daily, three times daily, once every other day, once weekly, twice weekly, three times weekly, once every other week, once every two weeks, once every three weeks, once every four weeks, *etc*., preferably once daily or twice daily.

In some embodiments of the present disclosure, the content of the compound represented by formula (I) or the pharmaceutically acceptable salt thereof in the medicament is 50 mg to 800 mg, such as 50 mg, 100 mg, 200 mg, 300 mg, 400 mg, 500 mg, 600 mg, 700 mg, or 800 mg, and the dosing frequency may be once daily or twice daily.

In some embodiments of the present disclosure, the content of compound B in the medicament is 100 mg, and the dosing frequency is once daily or twice daily.

In some embodiments of the present disclosure, the content of compound B in the medicament is 200 mg, and the dosing frequency is once daily or twice daily.

In some embodiments of the present disclosure, the content of compound B in the medicament is 400 mg, and the dosing frequency is once daily or twice daily.

In some embodiments of the present disclosure, the content of compound B in the medicament is 400 mg, and the dosing frequency is twice daily.

In some embodiments of the present disclosure, the content of compound B in the medicament is 600 mg, and the dosing frequency is once daily or twice daily.

In some embodiments of the present disclosure, the content of compound B in the medicament is 600 mg, and the dosing frequency is twice daily.

In some embodiments of the present disclosure, the content of compound B in the medicament is 800 mg, and the dosing frequency is once daily or twice daily.

In some embodiments of the present disclosure, the medicament is administered at a dose (calculated based on the active ingredient) of 4 mg/kg to 20 mg/kg, and the dosing frequency may be once daily or twice daily.

In some embodiments of the present disclosure, the medicament is administered at a dose of 4 mg/kg to 6 mg/kg, and the dosing frequency is once daily or twice daily.

In some embodiments of the present disclosure, the medicament is administered at a dose of 6 mg/kg to 8 mg/kg, and the dosing frequency is once daily or twice daily.

In some embodiments of the present disclosure, the medicament is administered at a dose of 8 mg/kg to 10 mg/kg, and the dosing frequency is once daily or twice daily.

In some embodiments of the present disclosure, the medicament is administered at a daily dose of 400 mg/day, 600 mg/day, 800 mg/day, or 1200 mg/day.

In another aspect, the present disclosure provides a pharmaceutical composition comprising a therapeutically effective amount of the compound represented by formula (I) or the pharmaceutically acceptable salt thereof as described above, and one or more pharmaceutically acceptable carriers, for use in the manufacture of a medicament for treating and/or preventing a kidney disease, wherein the kidney disease is diabetic nephropathy or lupus nephritis.

In another aspect, the present disclosure provides a unit dose of a pharmaceutical composition, wherein each unit dose comprises 50 mg to 800 mg of the compound represented by formula (I) or the pharmaceutically acceptable salt thereof, and one or more pharmaceutically acceptable carriers, for use in the manufacture of a medicament for treating and/or preventing a kidney disease, wherein the kidney disease is diabetic nephropathy or lupus nephritis.

In some embodiments of the present disclosure, each unit dose comprises, for example, 50 mg, 100 mg, 200 mg, 300 mg, 400 mg, 500 mg, 600 mg, 700 mg, or 800 mg of the compound represented by formula (I) or the pharmaceutically acceptable salt thereof, and one or more pharmaceutically acceptable carriers, for use in the manufacture of a medicament for treating and/or preventing a kidney disease, wherein the kidney disease is diabetic nephropathy or lupus nephritis.

In some embodiments of the present disclosure, the mode of administration is selected from the group consisting of simultaneous administration, independent formulation with co-administration, and independent formulation with sequential administration.

In some embodiments of the present disclosure, the route of administration is oral administration, parenteral administration, or transdermal administration, preferably oral administration. The parenteral administration is injection, including, but not limited to, intravenous injection, subcutaneous injection, or intramuscular injection.

### Definition of Terms

The term "halogen" refers to fluorine, chlorine, bromine, and iodine.

The term "alkyl" should be understood to mean a linear or branched saturated monovalent hydrocarbon group. For example, "C₁₋₆ alkyl" refers to a linear or branched alkyl group having 1, 2, 3, 4, 5, or 6 carbon atoms. The alkyl group is, for example, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, or isomers thereof.

The term "alkoxy" refers to an alkyl-O- group, wherein the alkyl group is as defined above. For example, "C₁₋₆ alkoxy" refers to a linear or branched alkyl-O- group having 1, 2, 3, 4, 5, or 6 carbon atoms; for example, methoxy, ethoxy, propoxy, butoxy, pentoxy, or hexoxy, or isomers thereof.

The term "deuteroalkyl" refers to an alkyl group substituted by one or more deuterium atoms, wherein the alkyl group is as defined above.

The term "haloalkyl" refers to an alkyl group substituted by one or more halogen atoms, wherein the alkyl group is as defined above.

The term "deuteroalkoxy" refers to an alkoxy group substituted by one or more deuterium atoms, wherein the alkoxy group is as defined above.

The term "haloalkoxy" refers to an alkoxy group substituted by one or more halogen atoms, wherein the alkoxy group is as defined above.

The term "alkylene" refers to a saturated divalent hydrocarbon group obtained by removing two hydrogen atoms from a saturated linear or branched hydrocarbon group, which may contain 1 to 20 carbon atoms, preferably 1 to 6 (*e.g.*, 1, 2, 3, 4, 5, or 6) carbon atoms. Non-limiting examples include methylene (-CH₂-), ethylene (-CH₂CH₂-), *etc*. The alkylene group may be substituted or unsubstituted.

The term "cycloalkyl" should be understood to mean a saturated monovalent monocyclic, bicyclic (*e.g.*, fused, bridged, or spiro), or tricyclic hydrocarbon ring, preferably "C₃₋₁₀ cycloalkyl". The term "C₃₋₁₀ cycloalkyl" should be understood to mean a saturated monovalent monocyclic, bicyclic (*e.g.*, bridged or spiro), or tricyclic hydrocarbon ring having 3, 4, 5, 6, 7, 8, 9, or 10 carbon atoms. C₃₋₁₀ cycloalkyl may be a monocyclic hydrocarbon group, such as cyclopropyl, cyclobutyl, cyclopentyl, cyclohexyl, cycloheptyl, cyclooctyl, cyclononyl, or cyclodecyl; or a bicyclic hydrocarbon group, such as bornyl, indolyl, hexahydroindolyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl, 2,7-diazaspiro[3,5]nonyl, or 2,6-diazaspiro[3,4]octyl; or a tricyclic hydrocarbon group, such as adamantyl.

The term "heterocyclyl" refers to a saturated or unsaturated non-aromatic ring or ring system, such as a 3- to 10-membered heterocyclyl group, which is a 3-membered, 4-membered, 5-membered, 6-membered, or 7-membered monocyclic ring, or a 7-membered, 8-membered, 9-membered, or 10-membered bicyclic (*e.g.*, fused, bridged, or spiro) ring containing, for example, 1, 2, 3, 4, 5, or more heteroatoms selected from the group consisting of O, S, and N, wherein N and S may also optionally be oxidized to form various oxidation states, such as N-oxides, -S(O)-, or -S(O)₂-. In particular, the heterocyclyl group may include, but is not limited to: 4-membered rings, such as azetidinyl or oxetanyl; 5-membered rings, such as tetrahydrofuranyl, dioxolanyl, pyrrolidinyl, imidazolidinyl, pyrazolidinyl, or pyrrolinyl; or 6-membered rings, such as tetrahydropyranyl, piperidinyl, morpholinyl, dithianyl, thiomorpholinyl, piperazinyl, or trithianyl; or 7-membered rings, such as diazepanyl. Optionally, the heterocyclyl group may be benzo-fused.

The term "cycloalkyloxy" refers to a cycloalkyl-O- group, wherein the cycloalkyl group is as defined above.

The term "heterocyclyloxy" refers to a heterocyclyl-O- group, wherein the heterocyclyl group is as defined above.

The term "diabetic kidney disease (DKD)" refers to a chronic kidney disease caused by diabetes mellitus, which results from renal damage caused by chronic hyperglycemia, and the lesions may involve the entire kidney (including glomeruli, renal tubules, renal interstitium, and renal vasculature). Clinically, DKD is characterized by a persistent increase in urinary albumin excretion and/or a progressive decline in glomerular filtration rate (GFR). For example, the clinical features of DKD include increased urinary albumin excretion, decreased GFR, and marked histopathological changes, including thickening of the glomerular basement membrane, mesangial expansion, nodular sclerosis, interstitial fibrosis, and tubular atrophy, as well as interstitial infiltration and vascular lesions. The deposition of C3a and C5b-9 in the glomeruli and tubulointerstitium of patients with DKD is significantly higher than in healthy subjects.

The term "pharmaceutically acceptable" refers to molecular entities and compositions that are physiologically tolerable when administered to humans and generally do not produce allergic or similar adverse reactions, such as gastrointestinal discomfort or dizziness.

The term "effective amount" or "therapeutically effective amount" refers to an amount of the compound of the present disclosure sufficient to achieve the intended application (including, but not limited to, the treatment of diseases as defined below). The therapeutically effective amount may vary depending on factors such as the intended application (*in vitro* or *in vivo*), or the subject being treated and the disease condition, such as the weight and age of the subject, the severity of the disease condition, and the mode of administration, which can be readily determined by a person of ordinary skill in the art. The specific dosage will vary depending on factors such as the particular compound selected, the dosing regimen employed, whether the compound is administered in combination with other compounds, the timing of administration, the tissue to which the compound is administered, and the physical delivery system employed.

The term "pharmaceutically acceptable carrier" includes any and all solvents, dispersion media, coatings, surfactants, antioxidants, preservatives (*e.g.*, antibacterial agents, antifungal agents), isotonic agents, absorption delaying agents, salts, preservatives, pharmaceuticals, pharmaceutical stabilizers, binders, excipients, disintegrants, lubricants, sweeteners, flavoring agents, dyes, and the like, and combinations thereof, unless any conventional carrier is incompatible with the active ingredient, for use in therapeutic or pharmaceutical compositions.

The term "lupus nephritis (LN)" refers to a renal complication caused by systemic lupus erythematosus (SLE). For example, LN clinically manifests as abnormally elevated urinary protein levels. In patients with active lupus nephritis, plasma levels of C3a, C5a, and soluble C5b-9 are significantly increased.

Currently, based on pathological types, LN is divided into minimal mesangial LN (Class I), mesangial proliferative LN (Class II), focal LN (Class III), diffuse LN (Class IV), membranous LN (Class V), and sclerosing LN (Class VI).

Minimal mesangial LN (Class I): Glomeruli appear normal under light microscopy, but immune complex deposition in the mesangial area can be observed by immunofluorescence and/or electron microscopy.

Mesangial proliferative LN (Class II): Varying degrees of proliferation of pure mesangial cells are visible under light microscopy, with or without increased mesangial matrix, accompanied by immune complex deposition in the mesangial area; small amounts of subepithelial or subendothelial immune complex deposition can be observed by immunofluorescence and electron microscopy.

Focal LN (Class III): active (A) or inactive (C) lesions, characterized by focal involvement (affecting less than 50% of glomeruli) with segmental or global endocapillary proliferation, membranoproliferation, and moderate to severe mesangial proliferation, with or without crescent formation; typical focal subendothelial immune complex deposition is observed, with or without mesangial changes. Class III(A): active lesions, referred to as focal proliferative lupus nephritis. Class III(A/C): active and chronic lesions, referred to as focal proliferative and sclerosing lupus nephritis. Class III(C): chronic inactive lesions with glomerulosclerosis, referred to as focal sclerosing lupus nephritis.

Diffuse LN (Class IV): active or inactive lesions, characterized by diffuse involvement (affecting 50% or more of glomeruli) with segmental or global endocapillary proliferation, membranoproliferation, and moderate to severe mesangial proliferation, or crescentic glomerulonephritis; typical diffuse subendothelial immune complex deposition is observed, with or without mesangial lesions. Class IV lupus nephritis is further divided into two subtypes: Class IV-S lupus nephritis: segmental lesions in more than 50% of glomeruli; Class IV-G lupus nephritis: global lesions in more than 50% of glomeruli. If diffuse "wire-loop" lesions are present, LN is classified as Class IV diffuse lupus nephritis, even in cases with mild or no cellular proliferation.

Membranous LN (Class V): characterized by diffuse thickening of the glomerular basement membrane, with diffuse or segmental subepithelial immune complex deposition, with or without mesangial lesions. Class V membranous lupus nephritis may coexist with Class III or Class IV lesions, in which case a composite diagnosis should be made (*e.g.*, Class V + Class III, Class V + Class IV), and may progress to Class VI sclerosing lupus nephritis.

Sclerosing LN (Class VI): More than 90% of glomeruli exhibit global sclerosis, with no active lesions remaining.

### BRIEF DESCRIPTION OF THE DRAWINGS

FIG. 1 shows 24-hour urinary albumin levels at the study endpoint in an SD rat nephropathy model induced by combined administration of BSA/LPS/CCL4;
FIG. 2 shows deposition of C3 in glomeruli in an SD rat nephropathy model induced by combined administration of BSA/LPS/CCL4;
FIG. 3 shows deposition of C5b-9 in glomeruli in an SD rat nephropathy model induced by combined administration of BSA/LPS/CCL4.

### Beneficial Effects

The compounds of the present disclosure exhibit therapeutic effects on diabetic nephropathy and lupus nephritis.

### DETAILED DESCRIPTION OF THE PREFERRED EMBODIMENT

The technical solutions of the present disclosure will be further illustrated in detail with reference to the following specific examples. It should be understood that the following examples are merely exemplary illustrations and explanations of the present disclosure, and should not be construed as limiting the scope of the present disclosure. All techniques implemented based on the contents of the present disclosure are encompassed within the intended scope of the present disclosure.

Unless otherwise specified, the raw materials and reagents used in the following examples are commercially available or can be prepared by known methods.

### Preparation Example 1: Preparation of Compound A

### Reaction Scheme for the Synthesis of Compound A and Intermediate b

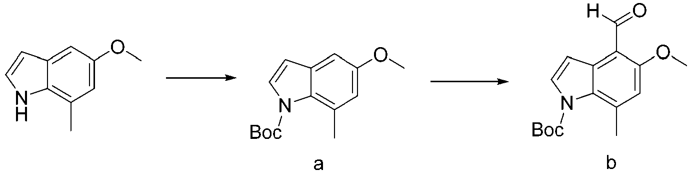

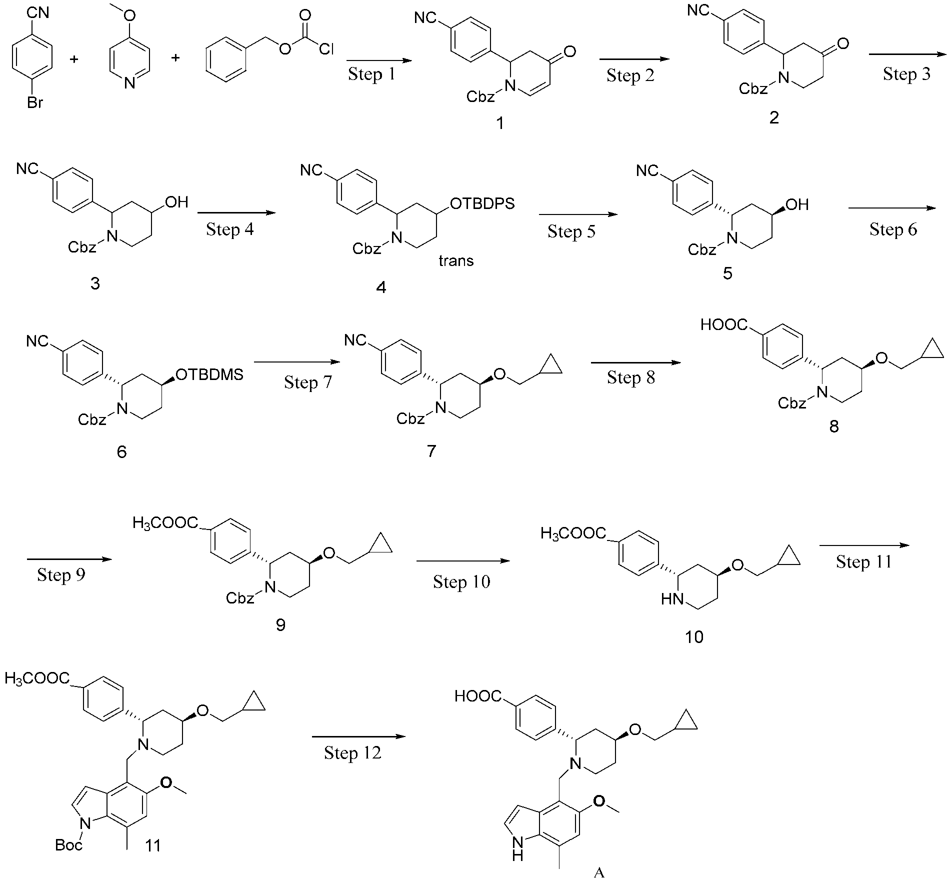

### Preparation of Intermediate b

Dichloromethane (50 mL), 5-methoxy-7-methyl-1*H*-indole (3 g), Boc anhydride (5.68 g), 4-dimethylaminopyridine (227 mg), and triethylamine (2.26 g) were sequentially added to a 250 mL single-necked flask. The reaction mixture was stirred at room temperature for 16 hours. After the reaction was completed, the reaction mixture was quenched with saturated ammonium chloride solution (5 mL) and extracted three times with dichloromethane (20 mL). The combined organic phases were washed with water (5 mL), dried over anhydrous sodium sulfate, and filtered. The filtrate was concentrated, and the residue was purified by column chromatography (petroleum ether: ethyl acetate = 10:1) to obtain intermediate a (4.6 g, yield: 94%). MS m/z (ESI): 262.0 [M+1].

Dichloromethane (80 mL), *N*-methylformanilide (3.8 g), and oxalyl chloride (3.6 g) were sequentially added to a 250 mL single-necked flask. The reaction mixture was stirred at room temperature for 3 hours. The reaction temperature was then lowered to -14°C, followed by the addition of intermediate a (2.5 g). The reaction system was naturally warmed to room temperature and stirred at room temperature for 1 hour. After the reaction was completed, the reaction mixture was poured into ice water (100 mL) and extracted three times with dichloromethane (100 mL). The combined organic phases were washed twice with water (10 mL), dried over anhydrous sodium sulfate, and filtered. The filtrate was concentrated, and the residue was purified by column chromatography (petroleum ether: ethyl acetate = 20:1) to obtain intermediate b (1.3 g, yield: 47%).

MS m/z (ESI): 290.0 [M+1]. ¹H NMR (400 MHz, CDCl₃) *δ* 10.65 (s, 1H), 7.65 (d, *J* = 3.4 Hz, 1H), 7.49 (d, *J* = 3.4 Hz, 1H), 6.76 (s, 1H), 3.98 (s, 3H), 2.70 (s, 3H), 1.65 (s, 9H).

### Preparation of Compound A

### Step 1:

Tetrahydrofuran (150 mL) and 4-bromobenzonitrile (50 g) were sequentially added to a 3 L three-necked flask. Isopropylmagnesium chloride-lithium chloride complex (1.3 M, 210 mL) was slowly added to the reaction system under a nitrogen atmosphere. The reaction mixture was stirred at room temperature for 2 hours. The reaction system was then diluted with anhydrous tetrahydrofuran (500 mL) and cooled to -5°C. 4-Methoxypyridine (25 mL) was added thereto, followed by the slow dropwise addition of benzyl chloroformate (35 mL) (maintaining the system temperature below 0°C). After the addition was completed, the reaction mixture was stirred at 0°C for 2 hours, then warmed to room temperature, and stirred at room temperature for another 16 hours. After the reaction was completed, the reaction mixture was added with 6 M hydrochloric acid (150 mL), stirred for 0.5 hours, diluted with water (1000 mL), and extracted twice with ethyl acetate (500 mL). The combined organic phases were washed with saturated brine (50 mL), dried over anhydrous sodium sulfate, and filtered. The filtrate was concentrated to obtain a crude product, which was purified by column chromatography (petroleum ether: ethyl acetate = 3:1 to 1:1) to obtain compound 1 (23 g, yield: 23%).

MS m/z (ESI): 333.0 [M+1].

### Step 2:

Compound 1 (28 g, obtained from two batches totaling 46 g in step 1), zinc powder (55 g), and acetic acid (200 mL) were sequentially added to a 500 mL single-necked flask. The reaction mixture was heated to 100°C and stirred at the same temperature for 16 hours. After the reaction was completed, the reaction mixture was filtered. The filtrate was diluted with water (500 mL) and extracted with ethyl acetate (500 mL). The organic phase was washed twice with saturated sodium bicarbonate aqueous solution (500 mL) and once with saturated brine (100 mL), dried over anhydrous sodium sulfate, and filtered. The filtrate was concentrated under reduced pressure to obtain compound 2 (26 g, yield: 73%).

MS m/z (ESI): 334.8 [M+1].

### Step 3:

Tetrahydrofuran (100 mL), ethanol (100 mL), and compound 2 (26 g) were sequentially added to a 500 mL single-necked flask, followed by the addition of sodium borohydride (2 g) in batches. The reaction mixture was stirred at room temperature for 2 hours. After the reaction was completed, the system was cooled to 0°C, and saturated ammonium chloride aqueous solution (30 mL) was added until no further temperature increase was observed. The reaction mixture was diluted with water (500 mL) and extracted twice with ethyl acetate (200 mL). The combined organic phases were washed with saturated brine (500 mL), dried over anhydrous sodium sulfate, and filtered. The filtrate was concentrated under reduced pressure to obtain compound 3 (25 g, yield: 76%).

MS m/z (ESI): 336.9 [M+1].

### Step 4:

Dichloromethane (200 mL) was added to a 500 mL single-necked flask, followed by the sequential addition of compound 3 (25 g), imidazole (6.6 g), and *tert*-butyldiphenylsilyl chloride (25 g). The reaction mixture was stirred at room temperature for 2 hours. After the reaction was completed, the reaction mixture was washed with water (500 mL), dried over anhydrous sodium sulfate, and filtered. The filtrate was concentrated under reduced pressure, and the residue was purified by column chromatography (petroleum ether: ethyl acetate = 10:1) to obtain compound 4 (5.7 g, yield: 13%, Rf = 0.55; *cis* isomer Rf = 0.50).

MS m/z (ESI): 597.0 [M+23].

### Step 5:

Compound 4 (5 g) and a solution of tetrabutylammonium fluoride in tetrahydrofuran (1 M, 30 mL) were sequentially added to a 250 mL single-necked flask. The reaction mixture was stirred at room temperature for 2 hours. After the reaction was completed, the reaction mixture was diluted with water (100 mL) and extracted three times with ethyl acetate (50 mL). The combined organic phases were washed with saturated brine (100 mL), dried over anhydrous sodium sulfate, and filtered. The filtrate was concentrated under reduced pressure, and the residue was purified by column chromatography (petroleum ether: ethyl acetate = 3:1 to 0:1) to obtain a racemic mixture, which was subjected to chiral separation by SFC (apparatus: SFC Thar prep 80; column: CHIRALPAK AD-H, 250 mm × 20 mm, 5 µm; modifier: 35% methanol (0.2% ammonia); column temperature: 40°C; column pressure: 60 bar; wavelength: 214/254 nm; flow rate: 40 g/min; Rt = 4.78 min) to obtain compound 5 (1.2 g, yield: 41%).

MS m/z (ESI): 358.8 [M+23].

### Step 6:

Imidazole (486 mg) and *tert*-butyldimethylsilyl chloride (593 mg) were added to a solution of compound 5 (1200 mg) in *N,N*-dimethylformamide (10 mL). The reaction mixture was stirred at room temperature for 2 hours. After the reaction was completed, the reaction mixture was diluted with water (100 mL) and extracted with ethyl acetate (50 mL). The organic phase was washed once with saturated brine (50 mL), dried over anhydrous sodium sulfate, and filtered. The filtrate was directly concentrated to obtain compound 6 (600 mg, yield: 90%).

MS m/z (ESI): 472.8 [M+23].

### Step 7:

Compound 6 (700 mg, obtained from two batches totaling 1.2 g in step 6) was added to dichloromethane (10 mL) at room temperature. Cyclopropanecarboxaldehyde (110 mg) and trimethylsilyl trifluoromethanesulfonate (35 mg) were added to the reaction mixture at - 78°C under a nitrogen atmosphere. The reaction system was maintained at -78°C and stirred for 1 hour, followed by the addition of triethylsilane (180 mg). The reaction mixture was naturally warmed to room temperature and stirred at the same temperature for another 16 hours. After the reaction was completed, the reaction mixture was quenched with saturated sodium bicarbonate aqueous solution (20 mL), diluted with water (10 mL), and extracted with dichloromethane (10 mL). The organic phase was washed once with water (10 mL), dried over anhydrous sodium sulfate, and filtered. The filtrate was concentrated, and the residue was purified by column chromatography (petroleum ether: ethyl acetate = 3:1) to obtain compound 7 (400 mg, yield: 46%).

MS m/z (ESI): 390.9 [M+1].

### Step 8:

Compound 7 (400 mg), isopropanol (2 mL), water (3 mL), and sodium hydroxide (400 mg) were sequentially added to a 50 mL single-necked flask. The reaction mixture was heated to 100°C and stirred at the same temperature for 16 hours. After the reaction was completed, the reaction mixture was added with dilute hydrochloric acid (1 M) to adjust the pH to 5 to 6 in an ice bath, diluted with water (5 mL), and extracted with ethyl acetate (5 mL). The organic phase was washed once with saturated brine (5 mL), dried over anhydrous sodium sulfate, and filtered. The filtrate was concentrated at 45°C to obtain compound 8 (200 mg, yield: 33%).

MS m/z (ESI): 431.8 [M+23].

### Step 9:

Potassium carbonate (135 mg) and iodomethane (140 mg) were added to a solution of compound 8 (200 mg) in acetonitrile (5 mL). The reaction mixture was heated to 50°C and stirred at the same temperature for 16 hours. After the reaction was completed, the reaction mixture was directly concentrated, and the residue was purified by column chromatography (petroleum ether: ethyl acetate = 3:1) to obtain compound 9 (180 mg, yield: 40%).

MS m/z (ESI): 445.8 [M+23].

### Step 10:

Palladium on carbon (50 mg) was added to a solution of compound 9 (180 mg) in tetrahydrofuran (3 mL). The reaction mixture was subjected to catalytic hydrogenation under a hydrogen atmosphere at room temperature for 2 hours. After the reaction was completed, the reaction mixture was filtered, and the filtrate was directly concentrated to obtain compound 10 (120 mg, yield: 54%).

MS m/z (ESI): 290.0 [M+1].

### Step 11:

Compound 10 (120 mg) was added to a solution of intermediate b (119 mg) in 1,2-dichloroethane (5 mL). The reaction mixture was stirred at room temperature for 8 hours, followed by the addition of sodium triacetoxyborohydride (261 mg), and stirred at room temperature for another 16 hours. After the reaction was completed, the reaction mixture was directly concentrated, and the residue was purified by column chromatography (dichloromethane: methanol = 20:1) to obtain compound 11 (200 mg, yield: 26%).

MS m/z (ESI): 562.8 [M+1].

### Step 12:

Methanol (2 mL), water (2 mL), compound 11 (200 mg), and sodium hydroxide (150 mg) were sequentially added to a 50 mL single-necked flask. The reaction mixture was heated to 75°C and stirred at the same temperature for 3 hours. After the reaction was completed, the reaction mixture was added with dilute hydrochloric acid (1 M) to adjust the pH to 7 in an ice bath, and then directly concentrated under reduced pressure. The residue was purified by preparative HPLC (column: Gemini-C18, 150 × 21.2 mm, 5 µm; mobile phase: acetonitrile-water (0.1% formic acid); gradient: 20 to 40%) to obtain compound A (30.6 mg, yield: 18%; containing 0.5 equivalents of formic acid).

MS m/z (ESI): 448.9 [M+1].

¹H NMR (400 MHz, CD₃OD): δ 8.18 (d, *J* = 7.7 Hz, 2H), 7.69 (d, *J* = 7.7 Hz, 2H), 7.32 (s, 1H), 6.76 (s, 1H), 6.34 (s, 1H), 4.88-4.61 (m, 1H), 4.44-4.07 (m, 2H), 3.95-3.81 (m, 1H), 3.75 (s, 3H), 3.63-3.47 (m, 1H), 3.46-3.33 (m, 3H), 2.50 (s, 3H), 2.35-2.14 (m, 2H), 2.13-1.94 (m, 2H), 1.23-1.04 (m, 1H), 0.58 (d, *J* = 7.2 Hz, 2H), 0.28 (d, *J* = 3.8 Hz, 2H).

Unless otherwise specified, compound A mentioned below was prepared according to the method described above or by repeating the method described above.

### Preparation Example 2: Preparation of Monohydrochloride Salt of Compound A

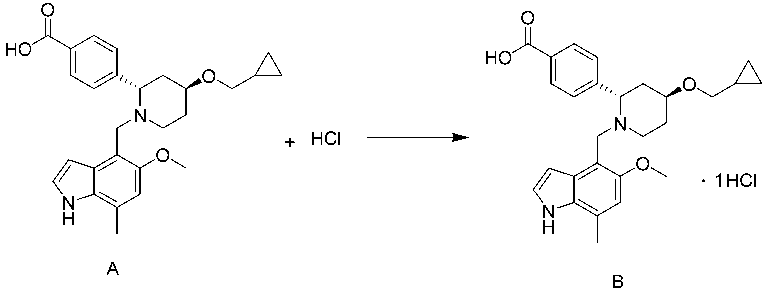

After compound A was prepared in multiple batches, 400 mg of compound A was added to 8 mL of isopropanol, and the mixture was heated at 50°C until complete dissolution. 460 µL of a solution of hydrochloric acid in isopropanol (2 mol/L) was slowly added dropwise thereto, and the reaction mixture was stirred for 0.5 hours. Subsequently, 8 mL of *n*-heptane was added thereto, and the reaction mixture was stirred for another 2 hours. After filtration, the filter cake was dried under reduced pressure at 50°C to obtain 390 mg of a monohydrochloride salt of compound A (compound B), with a yield of 90%.

### Test Example 1: In Vitro Cell Assay

The HK-2 cell line, derived from immortalized human renal proximal tubule epithelial cells (PTECs), was purchased from ATCC. HK-2 cells were cultured in DMEM/F12 medium (Gibco, 10565042) supplemented with 10% fetal bovine serum (Gibco, 10099-141C) and 1% penicillin/streptomycin (Gibco, 15140122). HK-2 cells were seeded into a 6-well plate (Corning, 3516) at a density of 1 × 10⁵ cells per well. When the cell confluence reached 70%, the cells were stimulated with gradient concentrations of the test compounds in the presence of High-Glucose (final concentration: 30 mmol/L; MilliporeSigma) for 24 hours. Cells were then harvested for real-time quantitative PCR analysis of mRNA expression levels of IL-1β, CFB, and TNF-α. Intracellular ROS (Beyotime Biotechnology, S0033S) and SOD (Beyotime Biotechnology, S0101S) levels were measured using assay kits. All procedures were performed according to the reagent manuals.

Total RNA was extracted from cells using the RNA Easy Fast Total RNA Extraction Kit (Tiangen Biotech, DP451). The primers used for mRNA assay are shown in the table. 2 µg of total RNA was reverse transcribed into cDNA using the High-Capacity cDNA Reverse Transcription Kit (Applied Biosystems, 4368814). Real-time RT-qPCR analysis was performed on a Bio-Rad CFX96 system using SYBR Green Master Mix (Applied Biosystems, A46012).

### RT-qPCR primer sequences

| Human | Forward primer | Reverse primer |
|---|---|---|
| β-actin | CCCTGGAGAAGAGCTACGAG | TCCATGCCCAGGAAGGAAG |
| CFB | CCATTGGCACAAGGAAGGTG | AAGGAGTCTTGGCAGGAAGG |
| IL-1β | AGCAACAAGTGGTGTTCTCC | CCAGCTGTAGAGTGGGCTTA |
| TNF-α | CTGCACTTTGGAGTGATCGG | AGGGTTTGCTACAACATGGG |

### Test Example 2: In Vivo Assay

Black and Tan Brachyury (BTBR) mice carrying the leptin deficiency mutation (ob/ob) (BTBR Lep^{ob/ob}, hereinafter referred to as BTBR ob/ob mice) represent a DKD model characterized by obesity, hyperglycemia, and dyslipidemia.

During the experiment, all male BTBR ob/ob mice were housed under SPF conditions. At 8 to 12 weeks of age, BTBR ob/ob mice had developed proteinuria and glomerular injury. The diseased BTBR ob/ob mice were randomly assigned to several groups and administered the test compounds by oral gavage once daily for 4 to 6 weeks. At the end of the dosing period, the experiment was terminated. Prior to euthanasia, the mice were placed in metabolic cages to collect 24-hour urine samples for assessment of urinary albumin, urinary creatinine, and the urinary albumin-to-creatinine ratio (UACR). Blood samples were collected to measure indicators such as glucose, cholesterol, and triglyceride levels. The mice were euthanized by CO₂ inhalation, and kidneys were harvested for histopathological analysis, including assessment of renal inflammation, glomerular area, loss of podocytes, and tubulointerstitial injury.

### Test Example 3: In Vivo Assay

Male db/db mice aged 3 to 4 weeks were intraperitoneally injected with STZ at a dose of 50 mg/kg for four consecutive days. One week after STZ administration, elevated blood glucose levels were observed. The test compounds were then administered orally for a continuous period of 2 to 6 months. Urine samples were collected at months 2 to 6 for urinary biochemical analysis. The mice were then euthanized, and serum, urine, and kidney tissues were collected for blood and urinary biochemical analysis and renal histopathological analysis.

Conclusion: Compound B was capable of reducing urinary albumin levels, UACR (urinary albumin-to-creatinine ratio), and complement deposition in kidney tissues in DKD model mice.

### Test Example 4: In Vivo Assay

Male db/db mice aged 6 to 9 weeks were grouped after their blood glucose levels increased to 16.7 mmol/L. The test compounds were then administered orally for a continuous period of 2 to 6 months. Urine samples were collected at months 2 to 6 for urinary biochemical analysis. The mice were then euthanized, and serum, urine, and kidney tissues were collected for blood and urinary biochemical analysis and renal histopathological analysis.

Following treatment with the test compounds, mice in the treatment group exhibited significant improvements in renal function-related indicators, such as urinary protein levels and renal histopathological scores.

### Test Example 5: In Vivo Assay

Spontaneously diseased NZBWF1 mice aged 24 to 26 weeks were used in this experiment. The test compounds were administered by oral gavage (PO) once daily (QD or BID) for 8 to 12 weeks. During the experiment, body weight, proteinuria, and mortality were recorded weekly. Blood urea nitrogen (BUN) and creatinine levels in serum were measured using an automated analyzer with an optimized ultraviolet method. Total IgG and anti-double-stranded DNA (anti-dsDNA) autoantibody levels in serum were measured by ELISA before administration and every 4 weeks after administration. At the study endpoint, kidney tissues were collected for histopathological analysis.

### Test Example 6: In Vivo Assay

Spontaneously diseased MRL/lpr mice were used in this experiment. The test compounds were administered by oral gavage (PO) starting from weeks 13 to 15 of the model for a continuous period of 8 to 12 weeks. During the experiment, body weight, proteinuria, and mortality were recorded weekly. Blood urea nitrogen (BUN) and creatinine levels in serum were measured using an automated analyzer with an optimized ultraviolet method. Total IgG and anti-double-stranded DNA (anti-dsDNA) autoantibody levels in serum were measured by ELISA before administration and every 4 weeks after administration. At the study endpoint, kidney tissues were collected for histopathological analysis.

Conclusion: Compound B was capable of improving, to a certain extent, renal function-related urinary protein levels and renal histopathological scores in lupus nephritis model mice.

### Test Example 7: In Vivo Assay

**Experimental Method**: The efficacy of compound B was evaluated in an SD rat nephropathy model induced by combined administration of BSA/LPS/CCL4. In the animal nephropathy model induced by combined administration of BSA (bovine serum albumin)/LPS/CCL4, diseased animals exhibit significantly elevated urinary protein levels and increased complement activity, as evidenced by enhanced deposition of C5b-9 and C3 in kidney tissues. These features show a certain consistency with the disease states, characteristics, and indicators requiring improvement in DKD and LN. Therefore, the model was used to evaluate the efficacy of compound B of the present disclosure.

A total of 60 male Sprague Dawley rats were used in this experiment. Model induction was performed by unilateral nephrectomy followed by combined induction with BSA/LPS/CCL4. All treatments were administered orally. The normal control group (Group 1, G1) and the model group (Group 2, G2) were administered vehicle. Group 3 (G3) was administered dexamethasone at a dose of 0.3 mg/kg via oral gavage once daily. Group 4 (G4) was administered compound B at a dose of 5 mg/kg via oral gavage twice daily. Group 5 (G5) was administered compound B at a dose of 15 mg/kg via oral gavage twice daily. Group 6 (G6) was administered compound B at a dose of 50 mg/kg via oral gavage twice daily. Administration was initiated four weeks after combined induction with BSA/LPS/CCL4 and continued for a period of six weeks. The evaluated indicators included body weight, food intake, urine volume, urinary albumin levels, and renal histopathological and immunofluorescence analyses.

**Experimental Results**: Compared with the normal control group, the model group exhibited a significant decrease in body weight, as well as significant increases in urinary albumin concentration, 24-hour urinary albumin levels, and ACR; the percentage of inflammatory infiltration area and the number of glomerular mesangial cells in kidney tissues were significantly increased in the model group; the deposition levels of C3 and C5b-9 in the glomeruli of model animals were significantly elevated.

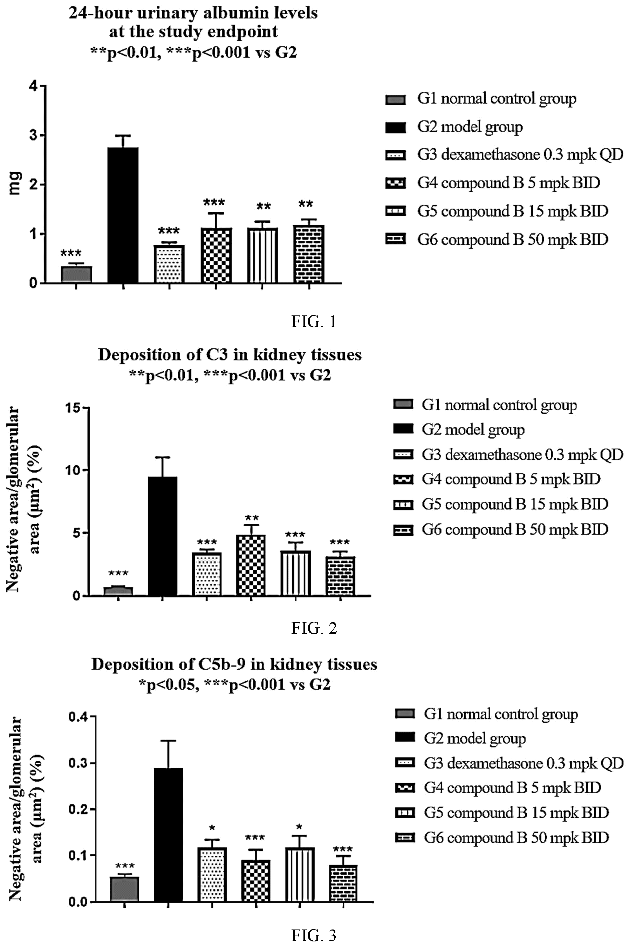

Compared with the model group, compound B dose-dependently and significantly reduced the urinary albumin concentration, 24-hour urinary albumin levels (see FIG. 1), and ACR in model animals at three weeks post-administration and at the study endpoint. Furthermore, compound B also significantly reduced the deposition levels of C3 and C5b-9 in the glomeruli of model animals (see FIG. 2 and FIG. 3).

### Test Example 8: A Clinical Trial Evaluating the Efficacy of the Compounds of the Present Disclosure in Patients with Diabetic Nephropathy

1. Experimental Design: This study adopted a multicenter, parallel, open-label design, consisting of three phases: a screening period (D-91 to D-1, a total of 13 weeks), an efficacy observation period (D1 to D84, a total of 12 weeks), and a follow-up period (D85 to D112, a total of 4 weeks).

The experimental drug was compound B:

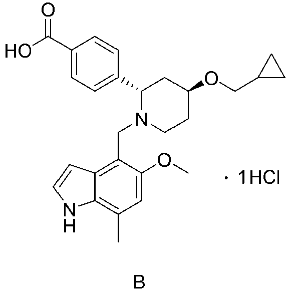

Compound B was administered at a dose of 400 mg BID, 600 mg QD, 800 mg QD, or 600 mg BID, either in the fasted state or in the fed state.

### 2. Experimental Objectives:

### 2.1 Primary Objective:

To evaluate the efficacy of compound B in patients with diabetic nephropathy at Day 84, as measured by the change from baseline in the urinary protein-to-creatinine ratio (UPCR) in first morning urine.

### 2.2 Secondary Objectives

2.2.1 To evaluate the efficacy of compound B in patients with diabetic nephropathy at D7, D14, D28, D42, D56, and D70, as measured by the change from baseline in the UPCR in first morning urine.

2.2.2 To evaluate the efficacy of compound B in patients with diabetic nephropathy at D7, D14, D28, D42, D56, D70, and D84, as measured by the change from baseline in the urinary albumin-to-creatinine ratio (UACR) in first morning urine.

2.2.3 To evaluate the efficacy of compound B in patients with diabetic nephropathy at D28, D56, and D84, as measured by the change from baseline in the estimated glomerular filtration rate (eGFR).

### 3. Inclusion Criteria:

3.1 Diagnosis of T2DKD confirmed by renal biopsy within one year prior to screening. If the most recent renal biopsy confirming the diagnosis was performed more than one year prior to screening, a renal biopsy may be performed at any time during the screening period to confirm the diagnosis of diabetic nephropathy and to assist in subject enrollment.

3.2 Urinary albumin-to-creatinine ratio (UACR) of 300 mg/g or more and less than 1500 mg/g during the screening period.

### 4. Exclusion Criteria:

4.1 eGFR of less than 30 mL/min/1.73 m² within four weeks prior to screening or during the screening period.

4.2 Diagnosis of rapidly progressive crescentic glomerulonephritis within one year prior to screening, defined as a 50% decline in eGFR within three months and renal biopsy findings showing crescent formation in at least 50% of glomeruli.

Test Example 9: A Clinical Trial Evaluating the Efficacy of the Compounds of the Present Disclosure in Patients with Lupus Nephritis
1. Experimental Design: This study adopted a multicenter, parallel, open-label design, consisting of three phases: a screening period (D-91 to D-1, a total of 13 weeks), an efficacy observation period (D1 to D84, a total of 12 weeks), and a follow-up period (D85 to D112, a total of 4 weeks).

The experimental drug was compound B:

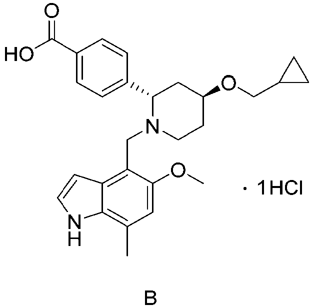

Compound B was administered at a dose of 400 mg BID, 600 mg QD, 800 mg QD, or 600 mg BID, either in the fasted state or in the fed state.

### 2. Experimental Objectives:

### 2.1 Primary Objective:

To evaluate the efficacy of compound B in patients with lupus nephritis at Day 84, as measured by the change from baseline in the urinary protein-to-creatinine ratio (UPCR) in first morning urine.

### 2.2 Secondary Objectives

2.2.1 To evaluate the efficacy of compound B in patients with lupus nephritis at D7, D14, D28, D42, D56, and D70, as measured by the change from baseline in the UPCR in first morning urine.

2.2.2 To evaluate the efficacy of compound B in patients with lupus nephritis at D7, D14, D28, D42, D56, D70, and D84, as measured by the change from baseline in the urinary albumin-to-creatinine ratio (UACR) in first morning urine.

2.2.3 To evaluate the efficacy of compound B in patients with lupus nephritis at D28, D56, and D84, as measured by the change from baseline in the estimated glomerular filtration rate (eGFR).

3. Inclusion Criteria:

3.1 Diagnosis of systemic lupus erythematosus (SLE) according to the 2019 European League Against Rheumatism/American College of Rheumatology (EULAR/ACR) classification criteria.

3.2 Diagnosis of active Class III or Class IV lupus nephritis confirmed by renal biopsy within three months prior to screening, with or without coexisting features of Class V lupus nephritis. If the most recent renal biopsy confirming the diagnosis was performed more than three months prior to screening, a renal biopsy may be performed at any time during the screening period to confirm the diagnosis of LN and to assist in subject enrollment.

3.3 Subjects with new-onset or relapsed LN, recurrence of LN following cyclophosphamide treatment, or recurrence of LN following mycophenolate mofetil (MMF) treatment may be enrolled in this study if the investigator, after thorough consideration in conjunction with the study protocol, deems it to have potential benefit for the patient.

### 4. Exclusion Criteria:

4.1 Subjects clinically diagnosed with lupus podocytopathy or lupus-associated thrombotic microangiopathy (TMA).

The embodiments of the present disclosure have been described above. However, the present disclosure is not limited to the aforementioned embodiments. Any modifications, equivalent substitutions, or improvements made within the spirit and principles of the present disclosure shall be included within the scope of protection of the present disclosure.

## Claims

1. Use of a compound represented by formula (I) or a pharmaceutically acceptable salt thereof in the manufacture of a medicament for treating and/or preventing a kidney disease, wherein the kidney disease is diabetic nephropathy or lupus nephritis, 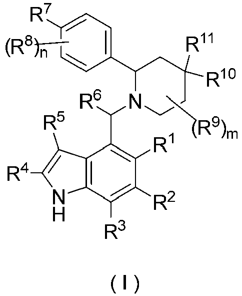 wherein
R¹ is selected from the group consisting of halogen, OH, CN, C₁₋₆ alkyl, C₃₋₁₀ cycloalkyl, C₁₋₆ alkoxy, C₁₋₆ deuteroalkoxy, C₃₋₁₀ cycloalkyloxy, C₁₋₆ haloalkyl, and C₁₋₆ haloalkoxy;
R² is selected from the group consisting of H, halogen, OH, CN, C₁₋₆ alkyl, C₃₋₁₀ cycloalkyl, C₁₋₆ alkoxy, C₁₋₆ haloalkyl, and C₁₋₆ haloalkoxy;
R³ is selected from the group consisting of H, halogen, OH, CN, C₁₋₆ alkyl, C₁₋₆ deuteroalkyl, C₃₋₁₀ cycloalkyl, C₁₋₆ alkoxy, C₃₋₁₀ cycloalkyloxy, NH₂, C₁₋₆ haloalkyl, and C₁₋₆ haloalkoxy;
R⁴ is selected from the group consisting of H, C₁₋₆ alkyl, and C₁₋₆ haloalkyl;
R⁵ is selected from the group consisting of H, halogen, and C₁₋₆ alkyl;
R⁶ is selected from the group consisting of H, halogen, OH, CN, and C₁₋₆ alkyl;
R⁷ is selected from the group consisting of -COOH and 5-membered heteroaryl;
R⁸ is selected from the group consisting of H, halogen, OH, CN, and C₁₋₆ alkyl;
R⁹ is selected from the group consisting of H, halogen, OH, CN, C₁₋₆ alkyl, and C₁₋₆ haloalkyl; or any two R⁹ together form a C₁₋₆ alkylene group;
R¹⁰ and R¹¹ are the same or different, and each independently selected from the group consisting of H, halogen, OH, CN, C₁₋₆ alkyl, C₃₋₁₀ cycloalkyl, 3- to 10-membered heterocyclyl, C₁₋₆ alkoxy, C₃₋₁₀ cycloalkyloxy, and 3- to 10-membered heterocyclyloxy, wherein the C₁₋₆ alkyl, C₃₋₁₀ cycloalkyl, 3- to 10-membered heterocyclyl, C₁₋₆ alkoxy, C₃₋₁₀ cycloalkyloxy, and 3- to 10-membered heterocyclyloxy are optionally substituted by one or more R^{a};
R^{a} is selected from the group consisting of halogen, OH, CN, C₁₋₆ alkyl, C₃₋₁₀ cycloalkyl, 3- to 10-membered heterocyclyl, and C₁₋₆ alkoxy, wherein the C₁₋₆ alkyl, C₃₋₁₀ cycloalkyl, 3- to 10-membered heterocyclyl, and C₁₋₆ alkoxy are optionally substituted by one or more substituents selected from the group consisting of halogen, OH, CN, and C₁₋₆ alkyl;
n is selected from the group consisting of 0, 1, 2, 3, and 4;
m is selected from the group consisting of 0, 1, 2, 3, 4, 5, and 6.

2. The use according to claim 1, wherein the compound represented by formula (I) is a compound represented by formula (II) as follows, 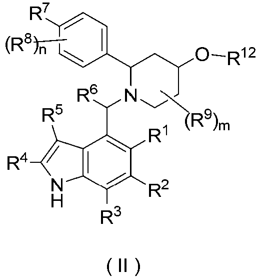 wherein R⁹ is selected from the group consisting of H, halogen, and C₁₋₆ alkyl; R¹² is selected from the group consisting of C₁₋₆ alkyl, C₃₋₆ cycloalkyl, and 3- to 6-membered heterocyclyl, wherein the C₁₋₆ alkyl, C₃₋₆ cycloalkyl, and 3- to 6-membered heterocyclyl are optionally substituted by one or more R^{a}; R^{a} is selected from the group consisting of halogen, C₃₋₆ cycloalkyl, and 3- to 6-membered heterocyclyl, wherein the C₃₋₆ cycloalkyl and 3- to 6-membered heterocyclyl are optionally substituted by one or more substituents selected from the group consisting of halogen, OH, CN, and C₁₋₆ alkyl; R¹ to R⁸, n, and m are as defined in claim 1; preferably, R¹ is C₁₋₆ alkoxy; R² is H; R³ is C₁₋₆ alkyl; R⁴ is H; R⁵ is H or halogen; R⁶ is H; R⁷ is -COOH; R⁸ is selected from the group consisting of H, halogen, and C₁₋₆ alkyl; R⁹ is selected from the group consisting of H, halogen, and C₁₋₆ alkyl; R¹² is C₁₋₆ alkyl, wherein the C₁₋₆ alkyl is optionally substituted by one or more R^{a}; R^{a} is selected from the group consisting of halogen, C₃₋₆ cycloalkyl, and 3- to 6-membered heterocyclyl, wherein the C₃₋₆ cycloalkyl and 3- to 6-membered heterocyclyl are optionally substituted by one or more substituents selected from the group consisting of halogen, OH, CN, and C₁₋₆ alkyl; n is selected from the group consisting of 0, 1, and 2; m is selected from the group consisting of 0, 1, and 2.

3. The use according to claim 1 or 2, wherein the compound represented by formula (I) may be selected from the group consisting of the following structures: 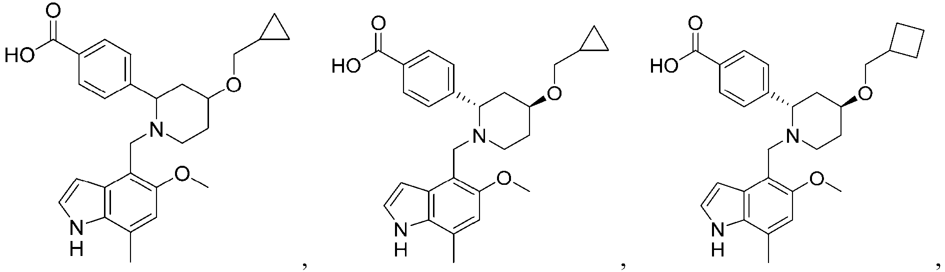 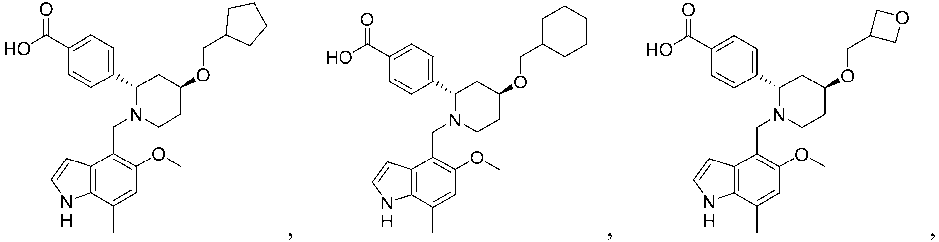 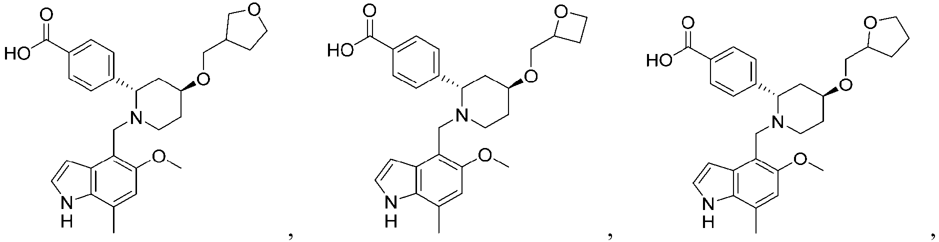 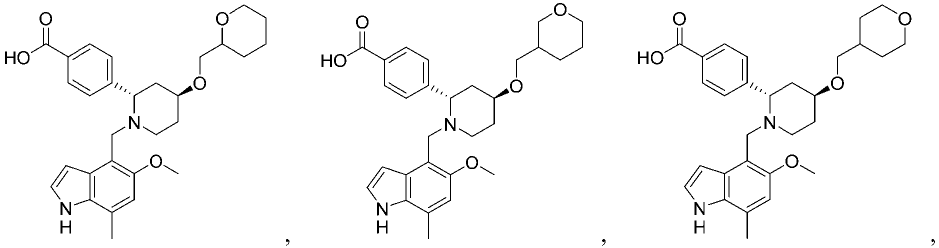 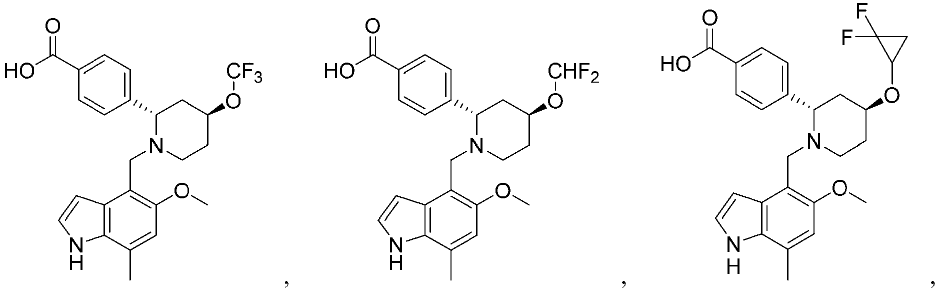 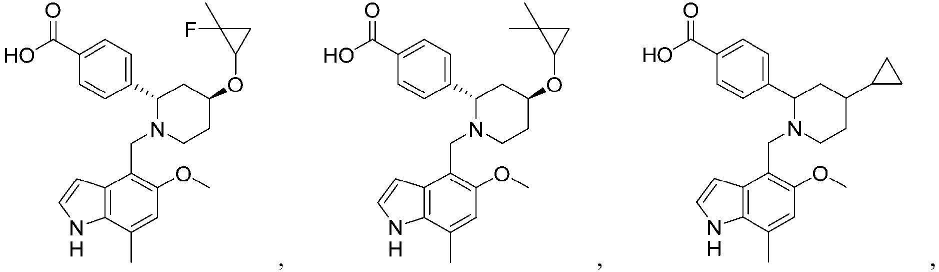 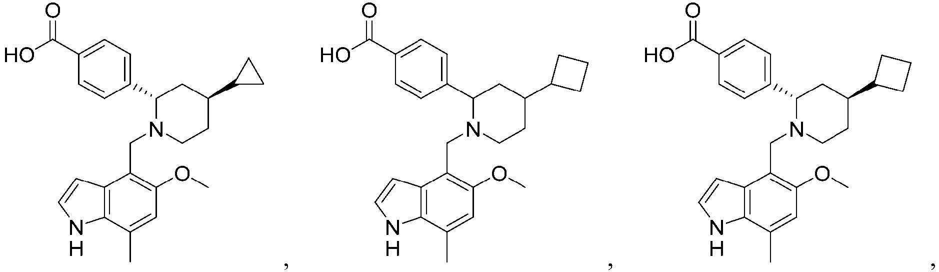 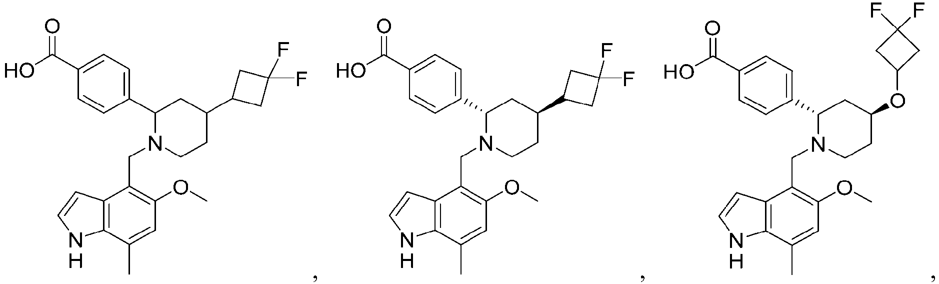 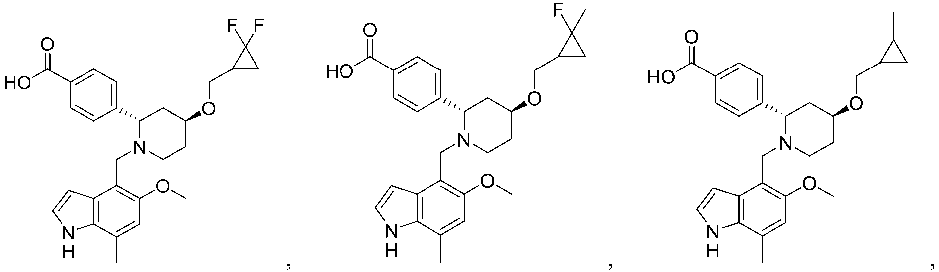 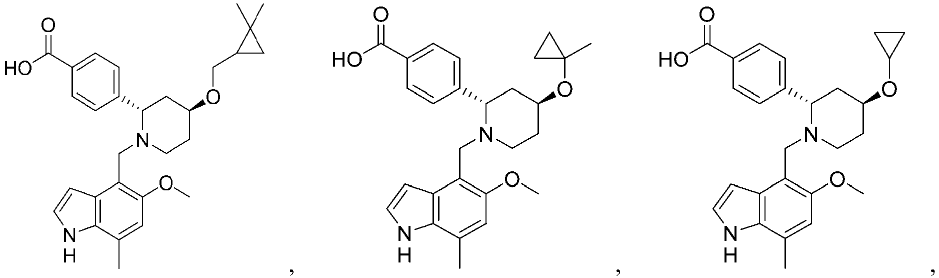 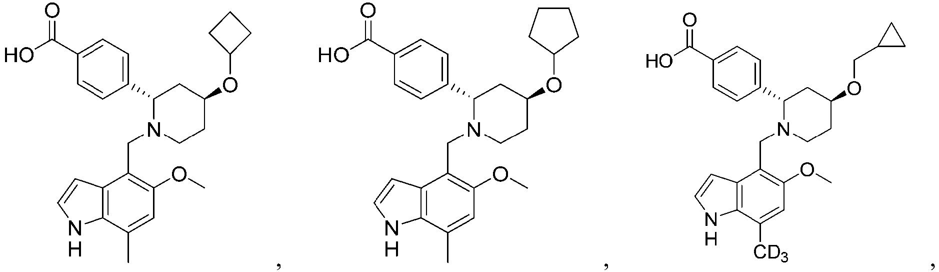 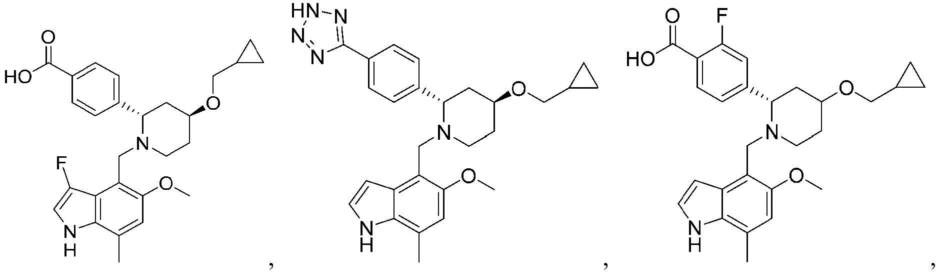 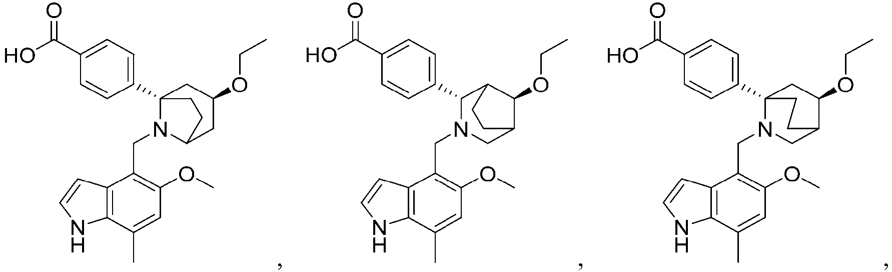 and

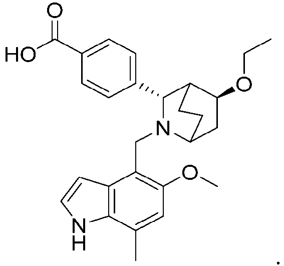

4. The use according to any one of claims 1 to 3, wherein the compound represented by formula (I) or the pharmaceutically acceptable salt thereof is compound A or compound B,

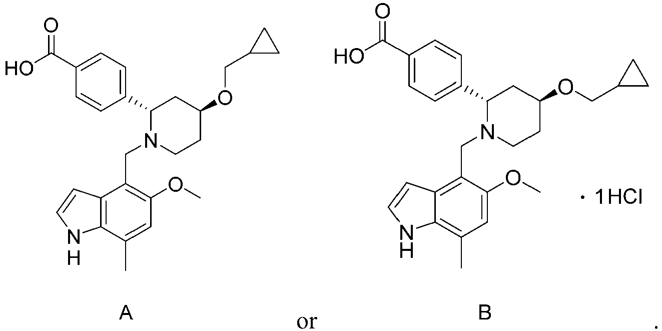

5. The use according to any one of claims 1 to 4, wherein the medicament is used for delaying progressive decline in renal function in a subject with diabetic nephropathy; or delaying progressive decline in glomerular filtration rate (GFR) in a subject with diabetic nephropathy; or improving a urinary protein-to-creatinine ratio (UPCR) and/or a urinary albumin-to-creatinine ratio (UACR) in a subject with diabetic nephropathy and lupus nephritis; preferably, the subject with diabetic nephropathy suffers from type 2 diabetes; preferably, the subject with diabetic nephropathy has a urinary albumin-to-creatinine ratio (UACR) of 300 mg/g or more and less than 1500 mg/g.

6. The use according to any one of claims 1 to 5, wherein the lupus nephritis is selected from the group consisting of minimal mesangial LN (Class I), mesangial proliferative LN (Class II), focal LN (Class III), diffuse LN (Class IV), membranous LN (Class V), and sclerosing LN (Class VI); or the lupus nephritis is new-onset or relapsed lupus nephritis; preferably, the relapsed lupus nephritis is lupus nephritis that has relapsed after treatment with cyclophosphamide or mycophenolate mofetil (MMF).

7. The use according to any one of claims 1 to 6, wherein the medicament is administered in an oral dosage form or an injectable dosage form; the medicament is administered at a dose (calculated based on the active ingredient) of 0.1 to 1000 mg; preferably 100 to 800 mg, more preferably 100 mg, 200 mg, 300 mg, 400 mg, 500 mg, or 600 mg; preferably, the dosing frequency of the medicament is selected from the group consisting of once daily, twice daily, three times daily, once every other day, once weekly, twice weekly, three times weekly, once every other week, once every two weeks, once every three weeks, and once every four weeks; more preferably, the dosing frequency of the medicament is selected from the group consisting of once daily and twice daily; more preferably, the medicament is administered at a daily dose of 400 mg/day, 600 mg/day, 800 mg/day, or 1200 mg/day.

8. The use according to any one of claims 1 to 7, wherein the medicament is a pharmaceutical composition comprising a therapeutically effective amount of the compound represented by formula (I) or the pharmaceutically acceptable salt thereof, and one or more pharmaceutically acceptable carriers; preferably, the medicament is a unit dose of a pharmaceutical composition, wherein each unit dose comprises 50 mg to 800 mg, for example 50 mg, 100 mg, 200 mg, 300 mg, or 400 mg of the compound represented by formula (I) or the pharmaceutically acceptable salt thereof, and one or more pharmaceutically acceptable carriers; preferably, the mode of administration of the medicament is selected from the group consisting of simultaneous administration, independent formulation with co-administration, and independent formulation with sequential administration; preferably, the route of administration of the medicament is oral administration, parenteral administration, or transdermal administration; the parenteral administration is injection, including, but not limited to, intravenous injection, subcutaneous injection, or intramuscular injection.

9. The use according to any one of claims 1 to 4, wherein the medicament is used for reducing 24-hour urinary albumin in a subject with diabetic nephropathy and lupus nephritis.

10. The use according to any one of claims 1 to 4, wherein the medicament is used for reducing the deposition of C3 and/or C5b-9 in the glomeruli of a subject with diabetic nephropathy and lupus nephritis.
